# EUROPEAN PATENT APPLICATION

(11) **EP 4 187 457 A1**
(43) Date of publication of application: **31.05.2023**
(21) Application number: 22204550.2
(22) Date of filing: 28.10.2022
(51) Int. Cl.: G06Q 10/06, G06N 7/00, G06N 20/00, G16H 50/20

(54) **DATA PROCESSING APPARATUS, DATA PROCESSING METHOD AND DATA PROCESSING PROGRAM**

(30) Priority: 30.11.2021 JP 2021193852
(71) Applicant: Hitachi, Ltd., Tokyo 100-8280 (JP)
(72) Inventor: YAMASHITA, Yasuho, Tokyo, 100-8280 (JP); SHIBAHARA, Takuma, Tokyo, 100-8280 (JP)
(74) Representative: Mewburn Ellis LLP

(57) **Abstract**

A data processing apparatus has a storage unit which stores an analysis target data group which has values of respective variables in a variable group and a value of an objective variable per analysis target and an element group that the variable group and each of one or more modulation method(s) for modulating the variable(s) are set as elements, a modulation unit which when the element which is selected from the element group is acquired, plans a modulation function of modulating the value of the variable which is contained in the acquired element on the basis of an action history which is the history of the acquired element and modulates the value of the variable per the analysis target on the basis of the modulation function and a generation unit which generates image data which gives a point of coordinates which are values of the modulation result and the objective variable to a coordinate space which is defined by a first axis which corresponds to a result of modulation by the modulation unit and a second axis which corresponds to the objective variable per the analysis target.

## Description

### CLAIM OF PRIORITY

The present application claims priority from Japanese patent application JP2021-193852 filed on November 30, 2021, the content of which is hereby incorporated by reference into this application.

### Technical Field

The present invention relates to a data processing apparatus, a data processing method and a data processing program for processing data.

### Background Art

To obtain an optimum numerical formula which expresses an objective variable by searching a numerical formula space which consists of a variable group and an arithmetic operation group which are prescribed is called symbolic regression. A model which expresses the objective variable can be obtained in an interpretable form which is called a numerical formula which consists of a prescribed arithmetic operation of a finite length by the symbolic regression.

Construction of a model which is configured by a human intelligible arithmetic operation and is expressed by a numerical formula of a formula length of the human intelligible extent is in demand in all fields which handle the model by the numerical formula such as science and engineering, medical and pharmaceutical sciences, economics and so forth. The symbolic regression by machine learning automatizes preparation of numerical formula models in such fields and contributes to advancement of learning.

Patent Literature 1 discloses a device and a method for generating and expanding a standard type formula which expresses characteristics of a given system. In this device and method, static and dynamic operations of a nonlinear electric circuit can be modeled and a progressive algorithm, simulated annealing and taboo searching can be used for searching for a standard formula.

Patent Literature 2 discloses a data processing device which facilitates analysis of a data group by a combination of a plurality of elements. This data processing device has a storage unit which stores an analysis target data group which has a factor and an objective variable per analysis target, a first modulation unit which modulates a first factor and outputs a first modulation result per the analysis target, a second modulation unit which modulates a second factor and outputs a second modulation result per the analysis target and a generation unit which generates first image data which gives a coordinate point which is a first modulation result from the first modulation unit and a second modulation result from the second modulation unit, per the analysis target, to a coordinate space which is defined by a first axis which corresponds to the first factor and a second axis which corresponds to the second factor and gives information which relates to the objective variable of the analysis target which corresponds to the coordinate point to the coordinate point.

### Citation List

### Patent Literature

Patent Literature 1: U.S. Unexamined Patent Application Publication No. 2017/0208548
Patent Literature 2: Japanese Patent Application Laid Open No. 2021-43626.

### Summary of Invention

### Technical Problem

Patent Literature 1 presents techniques of obtaining an optimum numerical formula in symbolic regression by using an evolutionary algorithm, or an annealing method, or Tabu search. However, these are the techniques which are said to be lower in accuracy than a technique of using deep learning, in general, in a regression problem. Accordingly, it is thought that the possibility that the optimum numerical formula can be obtained is lower than that of deep learning.

Although the technique in Patent Literature 2 performs the symbolic regression which uses reinforcement learning which is based on a deep learning model, there are limitations in form and length of the numerical formula.

The present invention aims to promote improvement of analytical accuracy.

### Solution to Problem

A data processing apparatus which becomes one profile of the invention which is disclosed in the present application has a storage unit which stores an analysis target data group which has values of respective variables in a variable group and a value of an objective variable per analysis target and an element group that the variable group and each of one or more modulation method(s) for modulating the variable(s) are set as elements, a modulation unit which when the element which is selected from the element group is acquired, plans a modulation function of modulating the value of the variable which is contained in the acquired element on the basis of an action history which is the history of the acquired element and modulates the value of the variable per the analysis target on the basis of the modulation function and a generation unit which generates image data which gives a point of coordinates which are values of the modulation result and the objective variable to a coordinate space which is defined by a first axis which corresponds to a result of modulation by the modulation unit and a second axis which corresponds to the objective variable per the analysis target.

### Effect of the Invention

According to a representative embodiment of the present invention, it aims to promote the improvement of the analytical accuracy. Issues, configurations and effects other than the aforementioned ones will become apparent from the following description of embodiments.

### Brief Description of Drawings

Fig. 1 is an explanatory diagram illustrating one example of data group analysis pertaining to an embodiment 1.
Fig. 2 is a block diagram illustrating a hardware configuration example of a data processing apparatus.
Fig. 3 is an explanatory diagram illustrating one example of an analysis target DB pertaining to the embodiment 1.
Fig. 4 is an explanatory diagram illustrating one example of a pattern table.
Fig. 5 is a block diagram illustrating a circuit configuration example of an image processing circuit.
Fig. 6 is an explanatory diagram illustrating one example of action history data.
Fig. 7 is a block diagram illustrating one configuration example of a controller which is illustrated in Fig. 5.
Fig. 8 is an explanatory diagram illustrating one example of an input/output screen which is displayed on an output device of the data processing apparatus pertaining to the embodiment 1.
Fig. 9 is a flowchart illustrating one detailed processing procedure example of image data generation processing by a modulation unit and an image generator.
Fig. 10 is a flowchart illustrating an analysis support process procedure example.
Fig. 11 is an explanatory diagram illustrating one example of a one-dimensional array.
Fig. 12 is an explanatory diagram illustrating one example of an analysis target DB pertaining to an embodiment 2.
Fig. 13 is an explanatory diagram illustrating one example of an input/output screen which is displayed on an output device of a data processing apparatus pertaining to the embodiment 2.

### Description of Embodiments

### [Embodiment 1]

In the following, one example of a data processing apparatus, a data processing method and a data processing program which pertain to the embodiment 1 will be described. In addition, in the embodiment 1, an analysis target data group is a combination of a value of each variable in a variable group and a value of an objective variable which indicates a one-year-later disease progressive state which is patient's 100 kinds of patient information which contains a body weight and a height as for each of 50 diabetic patients. Incidentally, the number of patients and the number of kinds of patient information are illustrative.

### <One Example of Analysis>

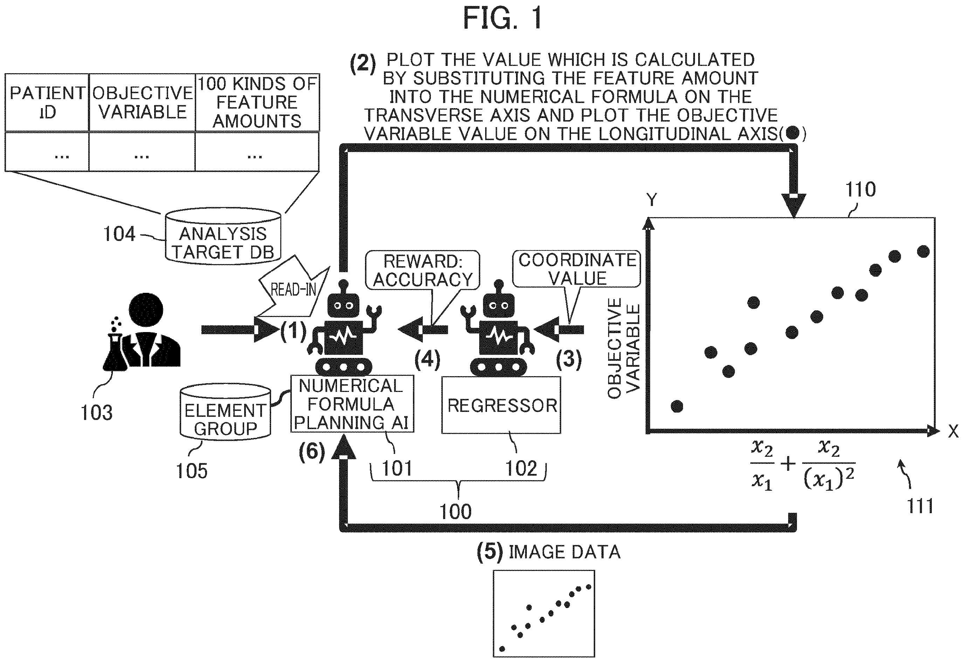

Fig. 1 is an explanatory diagram illustrating one example of analysis of a data group pertaining to the embodiment 1. A data processing apparatus 100 has a numerical formula planning AI (Artificial Intelligence) 101 and a regressor 102. The numerical formula planning AI 101 is, for example, a reinforcement learning type CNN (Convolutional Neural Network) which plans a numerical formula 111. The numerical formula 111 is a modulation function which modulates a variable group in order to make an objective variable interpretable by a defined operator.

The regressor (a regression model) 102 is an AI which inputs coordinates values on a coordinate space 110 which is spread by an X axis and a Y axis and outputs prediction accuracy as a reward to the numerical formula planning AI 101. A user 103 of the data processing apparatus 100 may be, for example, a medical doctor, a scholar and a researcher and may be a business person who provides an analysis service by the data processing apparatus 100.
(1) The user 103 selects an analysis target data group from an analysis target DB 104 which stores a patient-based analysis target data group and makes the numerical formula planning AI 101 read it. The analysis target data is, for example, a combination of a value of each variable (patient information) in the group of variables which are the above-described patient's 100 kinds of feature amounts and the objective variable.
(2) The numerical formula planning AI 101 selects a variable or a modulation method of modulating the variable from an element group 105. The numerical formula planning AI 101 selects, for example, "x1" from the element group 105 as the variable and selects "+" from the element group 105 as the modulation method. The modulation method is an operator which sets the variable as an operand or an indicator which indicates termination of an arithmetic operation.

The numerical formula planning AI 101 plans the X-axis numerical formula 111 which defines the coordinate space 110 on the basis of a history of the variable or the modulation method which is selected in (2). Then, the numerical formula planning AI 101 calculates the X-axis coordinate value by substituting the numerical formula which is specified by the history of the variable or the modulation method which is selected into the X-axis numerical formula 111 and substituting values of 100 kinds of the patient information which are feature amounts of the patient into the numerical formula concerned 111 and plots the value of the objective variable of the patient on the coordinate space 110 as a Y-axis coordinate value of the Y-axis which defines the coordinate space 110.

That is, as a scene that the numerical formula planning AI 101 sees in the reinforcement learning (a state which indicates what is going on as consequence of the action), they are plotted on the coordinate space 110 with the value of the objective variable of the patient being set as the Y-axis value and with a calculated value of the numerical formula 111 that the numerical formula planning AI 101 currently obtains being set as the X-axis value. Calculation of the X-axis coordinate value and the Y-axis coordinate value is executed per patient. The coordinate values which are plotted on the coordinate space 110 will be referred to as "patient data".

(3) The data processing apparatus 100 inputs the X-axis coordinate value in the patient data into the regressor 102.

(4) The regressor 102 calculates a linear regression equation which indicates the objective variable from the X-axis coordinate value in the patient data and calculates prediction accuracy thereof. Then, the regressor 102 outputs the calculated prediction accuracy to the numerical formula planning AI 101 as the reward in the reinforcement learning.

(5) In addition, the data processing apparatus 100 inputs image data I which is the coordinate space 110 that the patient data is plotted into the numerical formula planning AI 101, apart from (3).

(6) The numerical formula planning AI 101 executes a convolution operation over the reinforcement leaning type CNN in regard to the image data I in the coordinate space 110 by using the reward which is input in (4) and selects the next variable and modulation method from the element group 105. Then, the data processing apparatus 100 repeatedly executes (2) to (6).

The numerical formula planning AI repeatedly plans the numerical formula 111 while referring to the image data I in this way and thereby the user can obtain the numerical formula 111 which defines a new variable which highly correlates with the objective variable in the linear form. That is, searching for the numerical formula and determination of the optimum numerical formula for representing the objective variable become possible by using the reinforcement learning which is based on a deep learning model. Accordingly, the numerical formula is optimized and thereby improvement of the prediction accuracy of the objective variable can be promoted.

### <Hardware Configuration Example of Data Processing Apparatus 100>

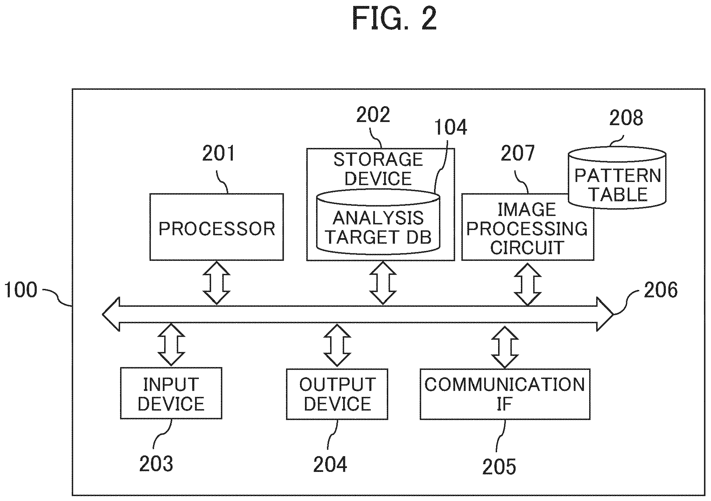

Fig. 2 is a block diagram illustrating a hardware configuration example of the data processing apparatus 100. The data processing apparatus 100 has a processor 2011, a storage device 202, an input device 203, an output device 204, a communication interface (communication IF) 205 and an image processing circuit 207. The processor 2011, the storage device 202, the input device 203, the output device 204, the communication IF 205 and the image processing circuit 207 are mutually connected via a bus 206.

The processor 201 controls the data processing apparatus 100. The storage device 202 serves as a working area of the processor 201. In addition, the storage device 202 is a non-transitory or transitory recording medium which stores various programs and data, the analysis target DB. As the storage device 202, there are, for example, a ROM (Read Only Memory), a RAM (Random Access Memory), an HDD (Hard Disk Drive) and a flash memory. The input device 203 inputs data. As the input data 203, there are, for example, a keyboard, a mouse, a touch panel, a numeric keypad, a scanner. The output device 204 outputs data. As the output device 204, there are, for example, a display, a printer. The communication IF 205 is connected with a network and transmits and receives data.

The image processing circuit 207 is a circuit configuration which executes image processing. The image processing circuit 207 executes processes in (1) to (6) which are illustrated in Fig. 1 with reference to a pattern table 208. The pattern table 208 is stored in, for example, a not illustrated storage region in the image processing circuit 207. Incidentally, although the image processing circuit 207 is realized by the circuit configuration, it may be also realized by making the processor 201 execute a program which is stored in the storage device 202.

### <Analysis Target DB 104>

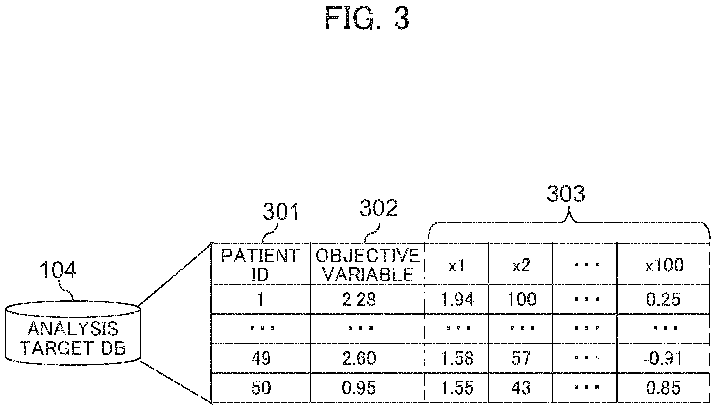

Fig. 3 is an explanatory diagram illustrating one example of the analysis target DB 104 pertaining to an embodiment 1. The analysis target DB 104 has Patient ID 301, Objective Variable 302 and Variable Group 303 as fields. A combination of values of respective fields on the same row becomes analysis target data on one patient.

Patient ID 301 is identification information for distinguishing one patient who is one example of the analysis target from other patients and values in Patient ID 301 are expressed by, for example, 1 to 50. Objective Variable 302 indicates values which indicate one-year-later disease progressive states of the patients. Variable Group 303 is, for example, a set of 100 kinds of variables. Each variable in Variable Group 303 indicates patient information. For example, a value of "1" in Patient ID 301 for a variable "x1" in Variable Group 303 is "1.94". That is, each entry of the analysis target DB 104 indicates the one-year-later disease progressive state of the patient which is specified by Variable Group 303.

### <Pattern Table 208>

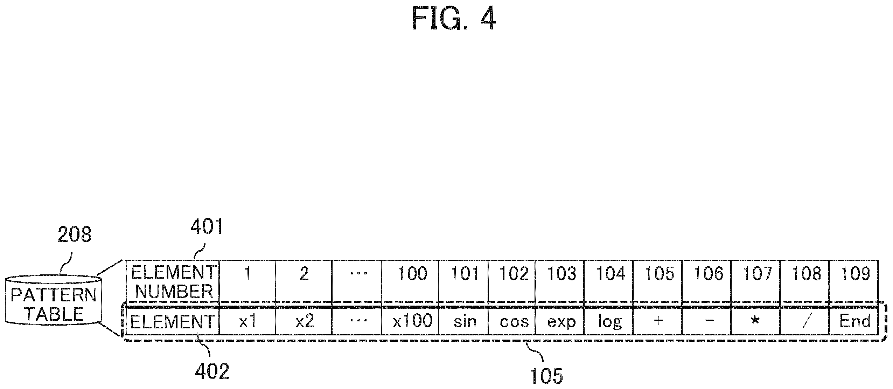

Fig. 4 is an explanatory diagram illustrating one example of the pattern table 208. The pattern table 208 is a table that the numerical formula planning AI 101 plans the numerical formula 111 and defines the element group 105 which is used for generation of a control signal for plotting the coordinate value on the coordinate space 110. Contents of the pattern table 208 are set in advance.

The pattern table 208 has Element Number 401 and Element 402 which corresponds to Element Number 401 as the fields. Element Number 401 is identification information for uniquely specifying a selection main constituent for selecting corresponding ones (x1, x2, log, +, End and so forth) in Element 402. Element 402 contains each variable in Variable Group 303, an operator that a value of the variable is set as an operand and an indicator which indicates end of the arithmetic operation. A unary operator and a polynomial operator are contained in the operator. For example, trigonometric functions (a sin function, a cos function, a tan function), an exponential function and a logarithmic function are contained in the unary operator. For example, four arithmetic operators are contained in the polynomial operator.

### <Configuration Example of Image Processing Circuit 207>

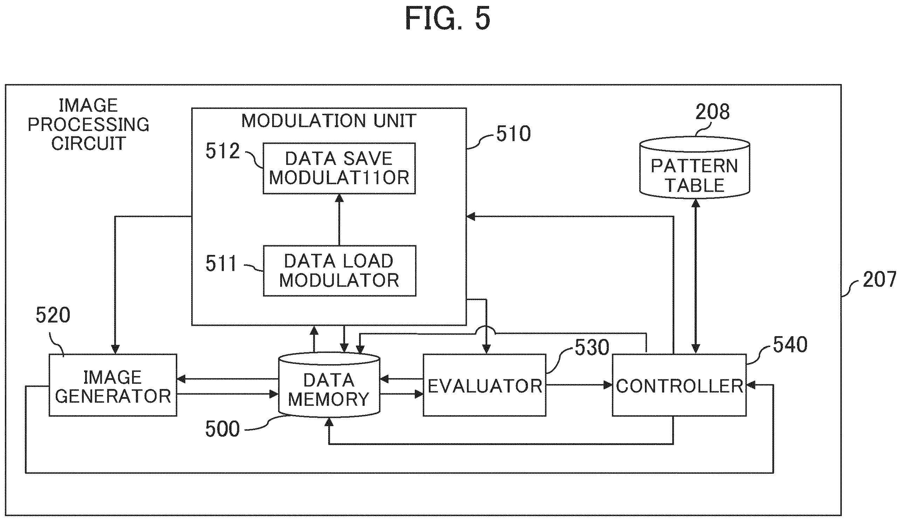

Fig. 5 is a block diagram illustrating one circuit configuration example of the image processing circuit 207. The image processing circuit 207 has a data memory 500, a modulation unit 510, an image generator 520, an evaluator 530, a controller 540, the pattern table 208.

All entries of the analysis target DB 104, that is, the analysis target data on each patient and the action history which is the history of the variable or the modulation method which is selected in the modulation unit 510 are written into the data memory 500 from the storage device 202.

The modulation unit 510 configures part of the numerical formula planning AI 101 which is illustrated in Fig. 1. The modulation unit 510 sets the variable or the modulation method in the numerical formula 111. The modulation unit 510 has a data load modulator 511 and a data save modulator 512.

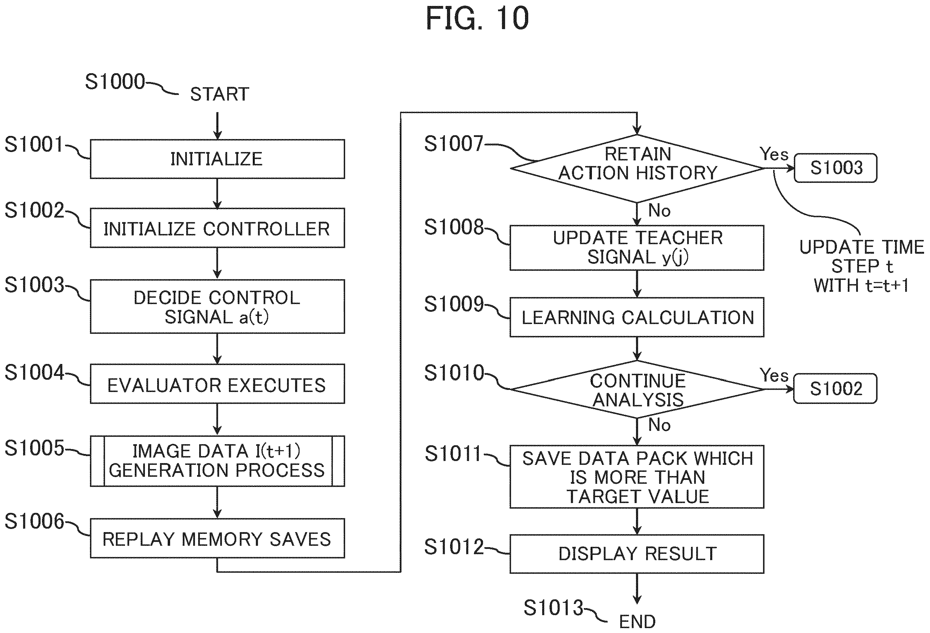

The data load modulator 511 acquires the one (the variable or the modulation method) in Element 402 which is contained in a control signal a(t) which is output from the controller 540 in a time step t (t is an integer which meets 0 ≤ t ≤ T, t=0 is an initial value, T is an integer which becomes the maximum value of t, for example, T = 30). The control signal a(t) contains the one in Element 402 which is randomly selected in the time step t. The control signal a(t) will be described later in step S1003 in Fig. 10. The data load modulator 511 may accept selection of the variable or the modulation method which is selected by the user.

The data load modulator 511 adds the acquired variable or modulation method to the column of the time step t in action history data which is stored in the data memory 500.

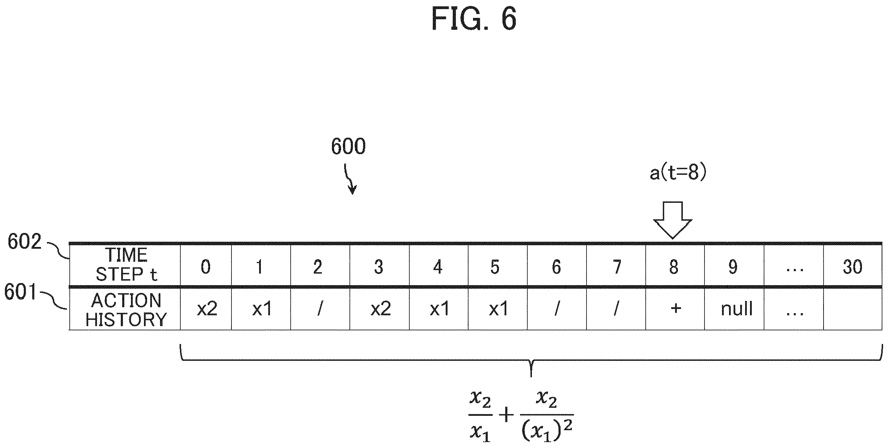

Fig. 6 is an explanatory diagram illustrating one example of action story data. The action history data 600 has Time Step 602 and Action History 601 in Time Step 602. The action history data 600 is sequence data which stores Action History 601 with Time Step 602 of t= 0, 1, 2, ... being set as columns and an initial value in Action History 601 is null. In the example which is illustrated in Fig. 6, the data load modulator 511 acquires the variable or the modulation method from a control signal a (t=8) by the modulation unit 510 in the latest time step in Time Step 602, t= 8, in this example and adds the acquired variable or modulation method to the column (in Time Step t) which corresponds to t=8 in Action History 601.

The data load modulator 511 acquires the numerical formula 111 by reading the sequence data in this Action History 601 by a specific numerical formula notation method, for example, Reverse Polish Notation. The data load modulator 511 calculates a vector which has X-axis coordinate values of all the patients when applying the value in Variable Group 303 in the analysis target data of the patients to numerical formula 111 in the variable which configures the numerical formula 111.

The data load modulator 511 may use a numerical formula notation method other than Reverse Polish Notation, for example, an infix notation method and Polish Notation. A vector value that the values in Variable Group 303 in the analysis target data on all the patients are applied to the numerical formula 111 is defined as a signal x'. For example, in a case where sequence data in Action History 601 is "x1, x2, +, x3, -", the numerical formula 111 which is obtained by reading the sequence data concerned by Reverse Polish Notation becomes "x1 + x2 - x3" and the signal x' becomes a vector which has the X-axis coordinate value of x' = x1 + x2 - x3 for all the patients.

There exists a case where it is impossible to convert data in Action History 601 to a meaningful numerical formula by the data load modulator 511. Specifically, for example, these are divided to a case (a case A) where the sequence data in Action History 601 is configured by only one or more variable(s) and any meaningful numerical formula cannot be obtained even when any variable or any modulation method is selected in the subsequent time steps and to a case (a case B) where the sequence data in Action History 601 is configured by only one or more modulation method(s) and there is the possibility that the meaningful numerical formula can be obtained by selecting an appropriate variable or modulation method in the subsequent time steps.

This case division can be decided depending on whether the meaningful variable can be obtained when generating the numerical formula by using, for example, Reverse Polish Notation, when filling actions in future time steps, that is, blank parts in Action History 601 with a certain variable (for example, the variable x1) one by one starting from more previous time steps or when filling them with a certain binary operator (for example, +) one by one starting from the more previous time steps.

In the case A, no useful numerical formula is obtained and it resultantly indicates that the action of generating a failed numerical formula is taken. Accordingly, it is so set that the signal x' takes a sufficiently large number, for example, 10¹⁰⁰ for all the patients.

In the case B, since it is thought that simply any useful numerical formula is not obtained temporarily in the current time step, the data load modulator 511 generates the signal x' which is based on the numerical formula 111 in an immediately previous time step t-1.

In the example which is illustrated in Fig. 6, the data load modulator 511 selects the variable or the modulation method in accordance with the control signal a (t=8) by the modulation unit 510 in the latest time step 602, t=8 in this example, updates it by adding the selected variable or modulation method to the column (in Time Step t) which corresponds to t=8 in Action History 601 and generates the numerical formula 111 on the basis of the updated Action History 601. The signal x' is generated by substituting the value in Variable Group 303 into this numerical formula.

Specifically, for example, the signal x' which is based on the action history data 600 in Fig. 6 becomes data that values of x1 and x2 in Variable Group 303 are substituted into the numerical formula 111 (x2/x1+x2/x1²) per patient ID 301 and becomes (100/1. 94+100/1. 94/1. 94, ...57/1. 58+57/1. 58/1. 58, 43/1. 55+43/1. 55/1. 55).

The data save modulator 512 saves the signal x' which is obtained by the data load modulator 511, the updated Action History 601 and numerical formula 111 into the data memory 500 and outputs them to the image generator 520.

In addition, when all the ones in Action History 601 are entirely filled by the load modulator 511, or when "End" is selected as the modulation method or when it is decided that the ones in Action History 601 apply to the above case A by the data load modulator 511, the modulation unit 510 sets a stop signal K(t) to K(t) = 1 and otherwise sets it to K(t) = 0. The stop signal K(t) is a signal for deciding resetting of the ones in Action History 601. In a case where K(t) = 1, the ones in Action History 601 and Time Step 602 are reset to initial values. In a case where K(t) = 0, the ones in Action History 601 and Time Step 602 are continuously retained.

The image generator 520 configures part of the numerical formula planning AI 101 which is illustrated in Fig. 1. The image generator 520 acquires the signal x' which is output from the modulation unit 510 and a value in Objective Variable 302 in the analysis target data which is saved in the data memory 500. The signal x' is a set of coordinate values (a one-dimensional vector) which are calculated from the numerical formula 111 per case. The image generator 520 plots the coordinate value that the value of the signal x' is set as the X-axis value and the value in Objective Variable 302 is set as the Y-axis value on the coordinate space 110 and thereby draws them as pixels which configure the image data I in the coordinate scape 110 that patient data is plotted.

The evaluator 530 has the regressor 102 which is illustrated in Fig. 1. The evaluator 530 acquires the signal x' which is output from the modulation unit 510 and the value in Objective Variable 302 from the data memory 500. The evaluator 530 calculates a statistical quantity r(t) in the time step t in accordance with the value in Objective Variable 302.

Specifically, for example, the evaluator 530 executes the regressor 102 and thereby calculates the statistical quantity r(t) which indicates the prediction accuracy for predicting the value in Objective Variable 302 of the patient. The statistical quantity r(t) corresponds to the reward of the reinforcement learning and become a result of evaluation of a way of selecting the numerical formula 111. For example, a determination coefficient R² and a mean square error MSE can be adopted as the statistical quantity r(t). That is, the evaluator 530 evaluates the way of selecting the signal x' which is the result of modulation on the basis of the numerical formula 111 which is obtained because that signal x' is selected.

The regressor 102 and, a logistic regression unit, a linear regression unit which work as regression calculation units, a neural network or a gradient boosting unit are loaded on the evaluator 530. The evaluator 530 saves the statistical quantity r(t) into the data memory 500 and outputs it to the controller 540.

The controller 540 configures part of the numerical formula planning AI 101 which is illustrated in Fig. 1. The controller 540 is a reinforcement leaning type CNN. The controller 540 acquires image data I (in the following, image data I (t)) of the time step t which is generated by the image generator. In addition, the controller 540 acquires the statistical quantity r(t) from the evaluator 530 as the reward of the reinforcement learning.

Then, the controller 540 controls the modulation unit 510. Specifically, for example, when the image data I(t) is input from the image generator 520, the controller 540 generates the control signal a(t) which controls the modulation unit 510 and controls generation of image data I(t+1) in the next time step (t+1).

### <Configuration of Controller 540>

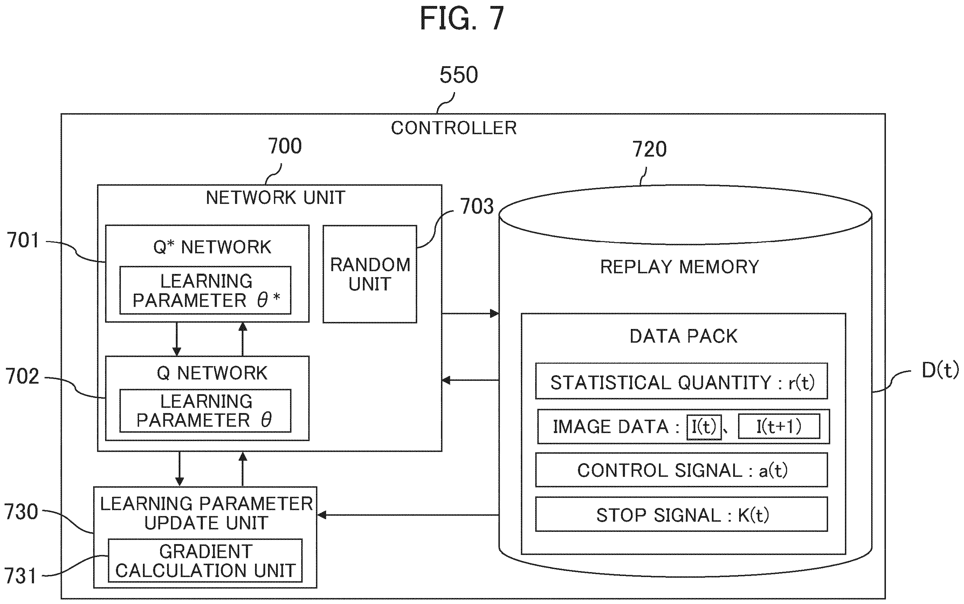

Fig. 7 is a block diagram illustrating one configuration example of the controller 540 which is illustrated in Fig. 5. The controller 540 has a network unit 700, a replay memory 720 and a learning parameter update unit 730. The network unit 700 has a Q* network 701, a Q network 702 and a random unit 703.

The Q* network 701 and the Q network 702 are action value functions of the same configuration for learning the control signal a(t) which is such an action of maximizing the value. The value, in this case, is an index value which indicates a height in correlation between a new variable which is obtained from the numerical formula 111 which is finally generated in the image data I(t) and the value in Objective Variable 302 by taking an action (planning the numerical formula 111) which is defined by the control signal a(t).

That is, the Q network 702 and the Q* network 701 select a maximum value in respective values in Element 402 in the element group 105 in the pattern table 208 when taking a certain action (the control signal a(t)) in a certain state (the image data I(t)).

Specifically, for example, the Q* network 701 is a deep reinforcement learning DQN (Deep Q-Network) which inputs the image data I(t) and outputs a one-dimensional array which indicates each value in Element 402 (the variable or the modulation method) in the control signal a(t) on the basis of a learning parameter θ*.

The Q network 702 is a deep reinforcement learning DQN which is the same as the Q* network 701 in configuration and obtains the value in Element 402 (the variable or the modulation method) which becomes a generation source of the image data I(t) with a learning parameter being defined as θ. The Q* network 701 selects an action which is the highest in the value of the image data I(t) which is obtained by the Q network 702, that is, the value in Element 402 in the pattern table 208.

The random unit 703 outputs a random number value which is more than 0 and less than 1 and which becomes a threshold value for deciding whether the action is randomly selected or is selected on the basis of the Q* network 701. The learning parameter update unit 730 has a gradient calculation unit 731. The learning parameter update unit 730 calculates a gradient g that the statistic quantity r(t) is considered as the reward by using the gradient calculation unit 731, adds the gradient g to the learning parameter θ and thereby updates the learning parameter θ.

The replay memory 720 stores a data pack D(t). The data pack D(t) contains the statistical quantity r(t), the image data I(t), I (t + 1), the control signal a(t) and the stop signal K(t) in the time step t. In a case of taking the action (the control signal a(t)) in the state of the time step t (the image data I (t)) by the data pack D(t), whether Action History 601 and the time step t are reset (the stop signal K(t)) is specified.

A configuration example of the Q* network 701 will be specifically described. Here, the configuration example of the Q* network 701 will be described by taking a case where the color image data I(t) of 84x84 pixels is input by way of example.

A first layer is a convolution network (a kernel: 8x8 pixels, a stride: 4, an activation function: ReLU). A second layer is a convolution network (the kernel: 4x4 pixels, the stride: 2, the activation function: ReLU). A third layer is a fully coupled network (the number of neurons: 256, the activation function: ReLU). In addition, an output layer is a fully coupled network and outputs a one-dimensional array z(t) which corresponds to the element column in the pattern table 208 as an output signal.

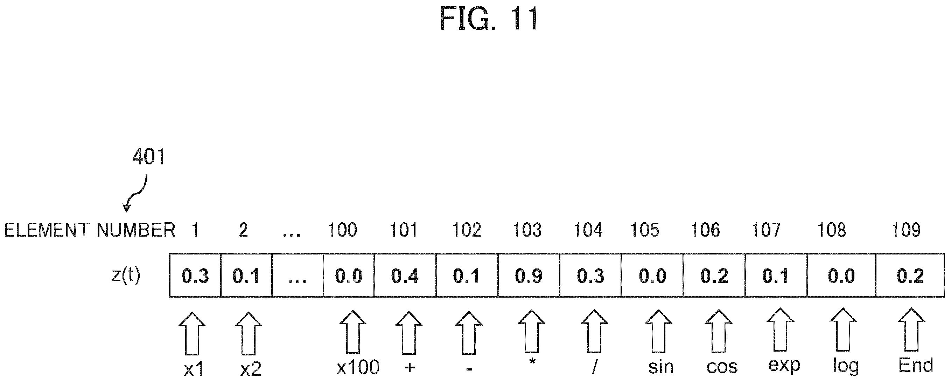

The one-dimensional array z(t) has values which are set in one-to-one correspondence with respective elements in Element 402 in the pattern table 208. That is, the one-dimensional array z(t) is an array which has values which correspond to 109 elements in Element 402 (see Fig. 11).

### <Input/Output Screen Example>

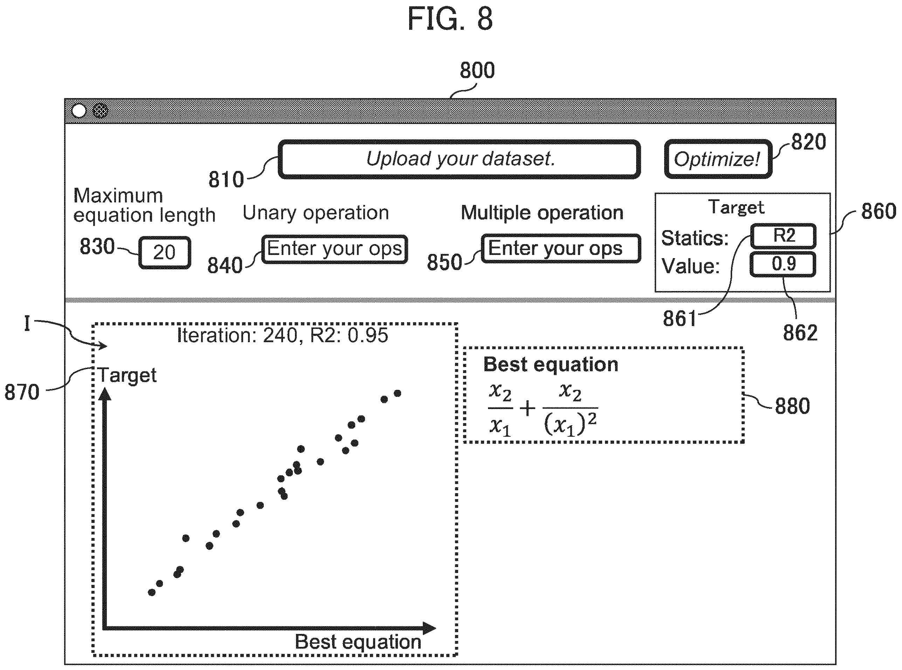

Fig. 8 is an explanatory diagram illustrating one example of an input/output screen which is displayed on the output device 204 of the data processing apparatus 100 pertaining the embodiment 1. An input/output screen 800 includes a load button 810, a start button 820, a numerical formula length input region 830, a unary operator input region 840, a polynomial operator input region 850, a target measurement input region 860, an image display region 870 and a numerical formula display region 880.

The load button 810 is a button for loading an entry of the analysis target DB 104 to the data memory 500 by pushing-down. The start button 820 is a button for starting image generation by pushing-down.

The numerical formula length input region 830 is adapted to input an upper limit of the length of the numerical formula which is generated. In a case where the numerical formula length input region 830 is empty, a numerical value of a default maximum numerical formula length (30 in this example) is automatically set.

Additional input of the unary operator which is one of the modulation methods in the data load modulator 511 into the unary operator input region 840 is possible. As the unary operator whose additional input into the unary operator input region 840 is possible, for example, a hyperbolic function and a constant multiple function which are not registered in the pattern table 208 can be additionally input thereinto. In a case where they are not additionally input thereinto, unary operators (for example, a sin function, a cos function, a tan function, an index function, a logarithm function) which are registered in the pattern table 208 are applied.

Additional input of the polynomial operator which is one of the modulation methods in the data load modulator 511 into the polynomial input region 850 is possible. As the polynomial operators whose additional input is possible, for example, additional input of a max function and a min function which are not registered in the pattern table 208 is possible. In a case where they are not additionally input, the polynomial operators (+, -, x, /) which are registered in the pattern table 208 are applied.

The target measure input region 860 includes a statistical quantity input region 861 and a target value input region 862. Kinds of the statistical quantities which are calculated by the learning parameter update unit 730 can be input into the statistical quantity input region 861. Specifically, for example, a statistical quantity for judging whether regression is good or bad such as a determination coefficient R2 can be selected. A target value of the statistical quantity which is input into the statistical quantity input region 861 can be input into the target value input region 862 (in Fig. 8, "0.8" by way of example).

The image data I which is generated by the image generator 520 is displayed in the image display region 870. The numerical formula 111 is displayed in the numerical formula display region 880.

Incidentally, the input/output screen 800 is displayed on, for example, a display which is one example of the output device 204 of the data processing apparatus 100. In addition, the input/output screen 800 may be displayed on a display of another computer concerned by transmitting information which relates to the input/output screen 800 from the communication IF 205 of the data processing apparatus 100 to that another computer which is connected with the communication IF to be communicable.

### <Image Data Generation Processing>

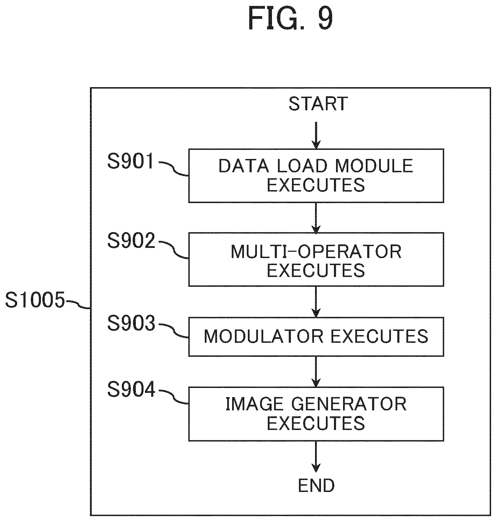

Fig. 9 is a flowchart illustrating a detailed process procedure example of the image data generation processing by the modulation unit 510 and the image generator 520. First, the data load modulator 511 in the modulation unit 510 executes a process (step S901). Specifically, for example, the data load modulator 511 selects one factor x1 from within the ones in Variable Group 303 which is stored in the data memory 500 by a control signal a(t+1) from the controller 540.

The data save modulator 512 calculates the numerical formula 111 in accordance with the action history data 600 which is updated by the control signal a(t+1), outputs the signal x', saves it in the data memory 500 and outputs it to the image generator 520 (step S903) .

The image generator 520 plots the patient data on the coordinate space 110 on the basis of the signal x' which is output from the modulation unit 510 and the value in Objective Variable 302 which is stored in the data memory 500 and generates the image data I (t+1) (step S904).

### <Analysis Processing Procedure Example>

Fig. 10 is a flowchart illustrating one analysis processing procedure example. Incidentally, before starting the processing, it is supposed that the entry of the analysis target DB 104 is loaded in the data memory 500 by pressing-down of the load button 810 in the input/output screen 800 in Fig. 8.

### [S1001]

The data processing apparatus 100 executes initialization (step S1001). Specifically, for example, the data processing apparatus 100 sets a calculation step m to m = 1. In addition, the data processing apparatus 100 initializes the learning parameter θ* of the Q* network 701 by random-weighting. In addition, the data processing apparatus 100 initializes the learning parameter θ of the Q network 701 by random-weighting.

### [S1002]

The data processing apparatus 100 initializes the controller 540 (step S1002). Specifically, for example, the data processing apparatus 100 makes all the columns in Action History 601 in the action history data 600 blank, sets the time step t to t=0 and sets image data I (t=0) to blank.

### [S1003]

The controller 540 determines the control signal a(t) of the time step t (step S1003). Specifically, for example, the random unit 703 outputs a random number value. When the random number value that the random unit 703 outputs is more than e (for example, e = 0.5), the controller 540 randomly selects one element in Element 402 from the pattern table 208 and determines the control signal a(t) with the selected element in Element 402. For example, when, in Element 402, the element which is randomly selected from the pattern table 208 is "/" which corresponds to the value "104" in Element Number 401, the controller 540 determines "/" as the control signal a(t).

On the other hand, when the random number value that the random unit 703 outputs is less than e, the controller 540 inputs the image data I(t) into the Q* network 701 in the network unit 700 and calculates a one-dimensional array z(t).

### <One-Dimensional Array z(t)>

Fig. 11 is an explanatory diagram illustrating one example of the one-dimensional array z(t). The one-dimensional array z(t) corresponds to 109 elements in Element Group 105 in the pattern table 208 and the array of 109 numerical values which are arrayed in the order of element numbers in Element Number 401. The magnitude of the numerical value indicates an action value of that element in Element 402.

The controller 540 selects one element in Element 402 in the pattern table 208 which corresponds to one element in Element 402 which reaches a maximum value, in the one-dimensional array z(t), and sets it as the control signal a(t). For example, in Fig. 11, the maximum value of the action value is "0.9" which corresponds to a value "103" in Element Number 401. In the pattern table 208, in Element 402, the element which corresponds to the value "103" in Element Number 401 is "*". The controller 540 sets the control signal a(t) in Fig. 7 to "*" which corresponds to the maximum value. The control signal a(t) which is higher in value can be selected by selecting the element in Element 402 that the action value reaches the maximum value in this way and thereby the controller 540 can take a more preferable action.

### [S1004]

Returning to Fig. 10, the evaluator 530 executes calculation of the statistical quantity r(t) of the time step t (step S1004). Specifically, for example, the evaluator 530 calculates the statistical quantity r(t) on the basis of the signal x' which is output from the modulation unit 510 and the value in Objective Variable 302 which is loaded from the data memory 500.

More specifically, the evaluator 530 executes the regressor 102 and thereby predicts the value in Objective Variable 302 per patient and calculates the statistical quantity r(t). The evaluator 530 saves the statistical quantity r(t) in the data memory 500 and outputs it to the controller 540.

### [S1005]

Next, the data processing apparatus 100 executes image data generation processing (in the following, image data I(t+1) generation processing) in the time step t+1 which is illustrated in Fig. 9 (step S1005). The image data I(t+1) generation processing (step S1005) generates the image data I(t+1) from the image generator 520 by giving the control signal a(t) which is determined in step S1004 to the modulation unit 510.

### [S1006]

Next, the network unit 700 saves a data pack D(t) that the statistical quantity r(t), Action History 601 of the time step t, the image data I(t), the image data I(t+1) and the stop signal K(t) are put together as one set of data in the replay memory 720 (step S1006) .

Specifically, for example, when the time step t is t=0, the image data I(t=0) which is generated in step S1002 and the image data I(t+1) which is generated in step S1005 are contained in a data pack D(0).

When the time step t is t=1, the image data I(t+1) which is generated in step S1005 and the image data I(t+2) which is generated in step S1005 when t=0 are contained in a data pack D(1).

In regard to t=2 and subsequent times, at the time step t, image data I((t-1) + 1) which is generated in step S1005 and the image data I(t+1) which is generated in step S1005 when t = t-1 are contained in the data pack D(t).

### [S1007]

Then, when K(t) = 0 (step S1007: Yes), Action History 601 of the time step t is retained and therefore the time step t is updated with t=t+1 and it returns to step S1003. On the other hand, when K(t) = 1 (step S1007: No), it shifts to step S1008.

### [S1008]

The learning parameter update unit 730 randomly loads J data packs D(1), ... D(j), ..., D(J) (j=1, ..., J) (in the following, a data pack group Ds) from the replay memory 720 and updates a teacher signal y(j) by the following formula (1) (step S1008) . Incidentally, in the embodiment 1, by way of example, the upper limit of J is defined to 100.

### [Numerical Formula 1] Formula (1)

In the above formula (1), γ is a discount rate and in the embodiment 1, γ = 0.998. A calculation process maxQ (I (j+1); θ) is a process of inputting image data I(j+1) into the Q network 702 in the network unit 700 and outputting the maximum value, that is, the maximum action value from within a one-dimensional array z(j) that the Q network 702 calculates by applying the learning parameter θ. "j+1" is the time step which comes after a time step t=j. For example, in a case where the one-dimensional array z(t) in Fig. 11 is the one-dimensional array z(j), the calculation process maxQ (I(j+1); θ) outputs the value "0.9" which corresponds to the value "103" in Element Number 401 as the maximum action value.

### [S1009]

Next, the learning parameter update unit 730 executes learning calculations (step S1009). Specifically, for example, the gradient calculation unit 731 outputs a gradient g in regard to the learning parameter θ by using the following formula (2), adds the gradient g to the learning parameter θ and thereby updates the learning parameter θ.

### [Numerical Formula 2]

The gradient g is the second term on the right side of the above formula (2). Thereby, the Q network 702 can generate the control signal a(t) which indicates such an action that the statistical quantity r(t), that is, the prediction accuracy of the value in Objective Variable 302 becomes high by the updated learning parameter θ that the statistical quantity r(t) which is the reward is considered.

In addition, in the learning calculation (step S1009), the learning parameter update unit 730 overwrites the updated learning parameter θ of the Q network 702 on a learning parameter θ* of the Q* network 701. That is, the learning parameter θ* reaches the value which is the same as that of the updated learning parameter θ. Thereby, the Q* network 701 can specify the action by which it can be expected that the action value, that is, the prediction accuracy of the value in Objective Variable 302 will become high.

### [S1010]

Next, when the statistical quantity r(t) falls below a target value which is input into the target value input region 862 and a calculation step m is less than a predetermined number of times M (step 1010: Yes), the data processing apparatus 100 returns to step S1002 in order to continue the analysis by the data processing apparatus 100 and updates the calculation step m with m = m+1. In the embodiment 1, it is defined that M = 1,000,000 times.

On the other hand, in a case where the statistical quantity f(t) becomes more than the target value which is input into the target value input region 862 or the calculation step m reaches the predetermined number of times M (step S1010: No), the data processing apparatus 100 shifts to step S1011.

### [S1011]

Next, the data processing apparatus 100 saves the data pack D(k) of a time step k that a statistical quantity r(k) becomes more than the target value in the data pack group Ds in the storage device 202 from the data memory 500 (step S1011). In a case where the data pack D(k) of the time step k that the statistical quantity r(k) becomes more than the target value is not present, it does not save the data pack D(k) in the storage device 202. In addition, in the case where the data pack D(k) of the time step k that the statistical quantity r(k) becomes more than the target value is not present, the data processing apparatus 100 may save the data pack D(k) of the time step k that the statistical quantity r(k) is maximized in the data pack group Ds in the storage device 202.

### [S1012]

Next, the data processing apparatus 100 displays a result of analysis (step S1012). Specifically, for example, the data processing apparatus 100 loads the data pack D(k) which is saved in the storage device 202, makes the modulation unit 510 execute numerical formula planning by using Action History 601 in the data pack D(k) and displays the planned numerical formula 111 in the numerical formula display region 880.

In addition, the data processing apparatus 100 displays image data I(k) and the statistical quantity r(k) in the data pack D(k) in the image display region 870. Incidentally, in a case where the data pack D(k) is not saved in the storage device 202, the data processing apparatus 100 may display a result of analysis which indicates a failure in analysis. Thereby, a series of processes is terminated (step S1013).

According to the embodiment 1, a new variable which linearly correlates with the objective variable can be automatically obtained in the form which is called the numerical formula which is configured by the variable in Variable Group 303 and the operator in this way. Thereby, improvement of the prediction accuracy of the value in Objective Variable 302 can be promoted.

### [Embodiment 2]

The embodiment 2 is an example of a case where Objective Variable 302 in the embodiment 1 is Categorical Variable. In the embodiment 2, in order to describe by focusing on different points between it and the embodiment 1, the same sign is assigned to the configuration which is the same as that in the embodiment 1 and description thereof is omitted.

### <Analysis Target DB 104>

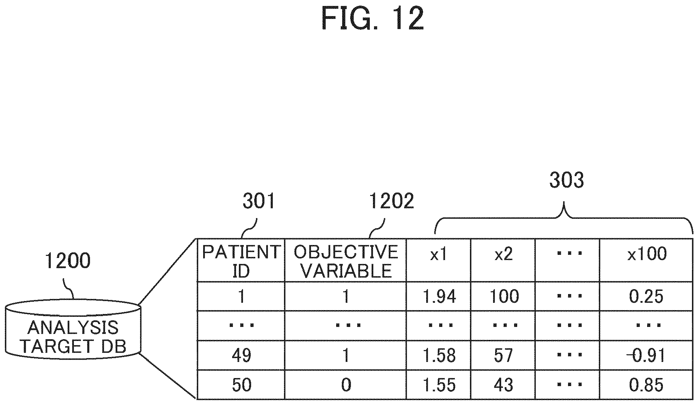

Fig. 12 is an explanatory diagram illustrating one example of the analysis target DB 104 pertaining to the embodiment 2. An analysis target DB 104 has Objective Variable 1202 which is Categorial Variable as the field, in place of Objective Variable 302. In Objective Variable 1202, a binary variable which indicates whether a disease state of each patient is deteriorated is stored as a value. Values in Objective Variable 1202 are, "1" indicates deterioration and "0" indicates no deterioration.

### <Input/Output Screen Example>

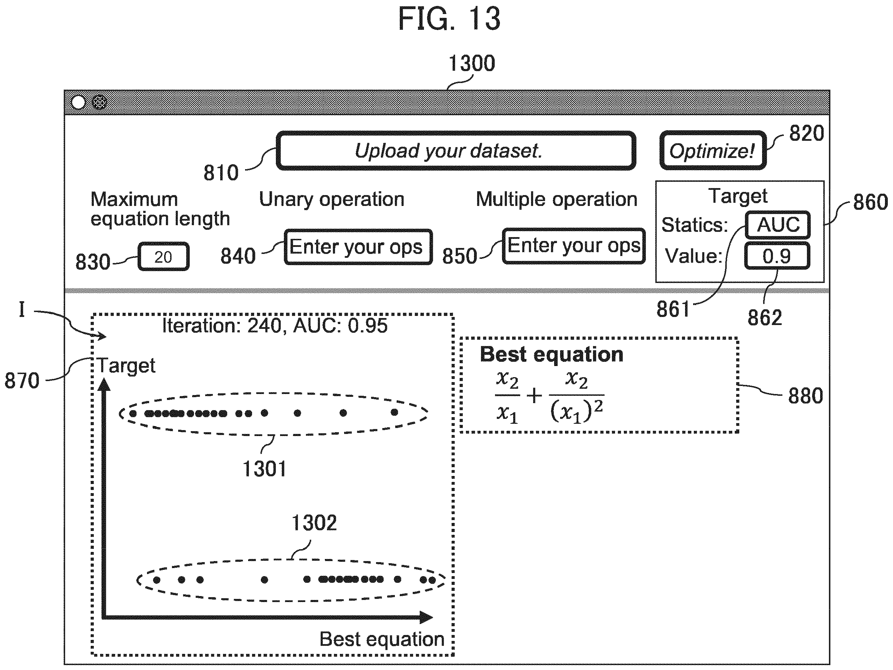

Fig. 13 is an explanatory diagram illustrating one example of an input/output screen which is displayed on the output device 204 of the data processing apparatus 100 pertaining to the embodiment 2. Since Objective Variable 1202 is Categorical Variable, on an input/output screen 1300, in the statistical quantity input region 861, for example, AUC (Area Under the Curve) can be selected as a statistical quantity r. In addition, target accuracy can be input into the target value input region 862 as a target value of the statistical quantity which is input into the statistical quantity input region 861 (in Fig. 13, "0.90" by way of example).

The image data I which is generated by the image generator 520 is displayed in the image display region 870. Since Objective Variable 1202 is Categorical Variable, the patient data group is classified into a disease state deterioration group 1301 that the value in Objective Variable 1202 is "1" (deterioration) and a disease state no-deterioration group 1302 that the value in Objective Valuable 1202 is "0" (no-deterioration) in the image data I.

Also in the embodiment 2, the numerical formula planning AI 101 repetitively plans the numerical formula 111 while referring to the image data I in this way and thereby the user can obtain the numerical formula 111 which defines a new variable which highly correlates with the objective variable in the linear form. That is, searching for a numerical formula for expressing the objective variable and determination of an optimum numerical formula become possible by using the reinforcement learning which is based on the deep learning model. Accordingly, the improvement of classification accuracy of the patient data group can be promoted according to the embodiment 2.

In addition, in the embodiment 1 and the embodiment 2, use of the patient information on the diabetic patients as the analysis target data is exemplified. However, the analysis target data is not limited to such biological information as above and, for example, it is also applicable to stocks. For example, the analysis target may be replaced with a brand of a company, the patient ID 301 may be replaced with ID of the brand and Variable Group 303 may be replaced with company information such as the net income, the number of employees, the sales figures and so forth of that company. In addition, in the case of the embodiment 1, Objective Variable 302 may be replaced with the stock price of the brand concerned. In addition, in the case of the embodiment 2, Objective Variable 302 (the quantitative variable) may be replaced with rising or falling of the brand concerned or purchase possibility/impossibility thereof.

From the above, according the embodiment 1 and the embodiment 2, the improvement of the analysis accuracy can be promoted.

In addition, the data processing apparatus 100 pertaining to the above embodiment 1 and second embodiment can be also configured as in the following (1) to (8).
(1) For example, the data processing apparatus 100 has the storage unit, the modulation unit 510 and the image generator 520. The analysis target DB 104 which is one example of the storage unit stores the analysis target data group (the analysis target DB 104) which has Variable Group 303 and Objective Variable 302, 1202 per analysis target and the pattern table 208 stores Variable Group 303 and the element group 105 that each of one or more modulation method(s) of modulating the variable is set as Element 402 which indicates the action of the controller 540.

When Element 402 which is selected from the element group 105 is acquired, the modulation unit 510 plans the numerical formula 111 as the modulation function of modulating the value of the variable in the acquired Element 402 on the basis of Action History 601 which is the history of the acquired Element 402 and modulates the value of the analysis-target-based variable on the basis of the numerical formula 111.

The image generator 520 generates the image data I that the coordinate point (the patient data) is given to the coordinate space 110 which is defined by the X-axis and the Y-axis per analysis target, with the modulation result (the signal x') from the modulation unit 510 and the value in Objective Variable 302 being set as the coordinate values of the X-axis and the Y-axis respectively.

(2) In addition, in the above (1), the data processing apparatus 100 has the controller 540. The controller 540 generates the control signal a(t) which selects Element 402 from the element group 105 in the pattern table 208 on the basis of the action history 601 and controls the modulation unit 510 on the basis of the control signal a(t).

Thereby, the controller 540 can plan the numerical formula 111 on the basis of Action History 601 and can output the coordinate value (the patient data). The image generator 520 can plot the coordinate value (the patient data) on the coordinate space 110 and thereby can generate the image data I(t).

(3) In addition, in the above (2), when the image data I(t) is generated by the image generator 520, the controller 540 may select Element 402 from the element group 105 and may freshly generate the control signal a(t).

Thereby, the image generator 520 can generate the image data I(t+1) that the action by the control signal a(t) is reflected and the controller 540 can take the next action in a state which is called the image data I(t+1) like this.

(4) In addition, in the above (3), the controller 540 may input the image data I(t+1) into the Q* network 701 which outputs the one-dimensional array z(t) which indicates the value in a case where the action which corresponds to each element in Element 402 in the pattern table 208 is taken in the state which is defined by the image data I(t+1) on the basis of the learning parameter θ*, may generate the control signal a(t) (an example: "*") which corresponds to a specific value (an example: 0.9) in the one-dimensional array z(t) which indicates each value in Element 402 which is output from the Q* network 701 and may control the modulation unit 510 on the basis of the control signal a(t).

Thereby, the image generator 520 can generate the image data I(t+1) that the action of the specific value by the control signal a(t) is reflected and the controller 540 can take the next action in the state which is called the image data I(t+1) like this.

(5) In addition, in the above (4), the specific value may be also set as a value which indicates a maximum value in the one-dimensional array z(t) which indicates each value in Element 402 in the pattern table 208.

Thereby, the image generator 520 can generate the image data I(t+1) that the action whose value by the control signal a(t) is maximum is reflected and the controller 540 can take the next action in the state which is called the image data I(t+1) like this.

(6) In addition, in the above (4), the data processing apparatus 100 has the replay memory 720 which saves the data pack group Ds and the evaluator 530 which evaluates the numerical formula 111 on the basis of the modulation result (the signal x') and the value in Objective Variable 302.

The controller 540 has the Q network 702 which outputs the one-dimensional array z(t) which indicates the value of each element in the pattern table 208 in a state which is defined by the image data on the basis of the learning parameter θ. The controller 540 may add the statistical quantity r(j) which is the result of evaluation of the numerical formula 111 by the evaluator 530 to the second output result of a case where the image data I(j+1) is input into the Q network 702 as the reward and thereby calculate the value of the action as a teacher signal y(j), and may update the learning parameter θ on the basis of the teacher signal y(j) and the first output result of a case where image data I(j) is input into the Q network 702 and may update the learning parameter θ* by the updated learning parameter θ.

Thereby, optimization of the Q* network 701 can be promoted and an element which is higher in value can be specified from the one-dimensional array z (t) that the Q* network 701 outputs. Accordingly, identification and regression analysis of the patient data group with a high accuracy by the devised numerical formula 111 become possible.

(7) In addition, in the above (1), the data processing apparatus 100 has the evaluator 530 and the output unit (the output device 204 or the communication IF 205). The evaluator 530 evaluates the numerical formula 111 on the basis of the modulation result (the value that a numerical value in the variable group of the patient data is substituted into the planned numerical formula 111) (the signal x') and the value in Objective Variable 302. In a case where the statistical quantity r(j) which is the evaluation result of the numerical formula 111 by the evaluator 530 is, for example, more than the target value which is input into the target value input region 862, the output unit may output the image data I(j) to be displayable.

Thereby, the data processing apparatus 100 can narrow down the numerical formulae 111 that the user 103 needs.

Incidentally, the present invention is not limited to the aforementioned embodiments and various modified examples and equivalent configurations within the gist of the appended Claims are included. For example, the aforementioned embodiments are described in detail in order to intelligibly describe the present invention and the present invention is not necessarily limited to the one which is equipped with all the configurations which are described. In addition, part of a configuration of a certain embodiment may be replaced with a configuration of another embodiment. In addition, the configuration of another embodiment may be added to the configuration of the certain embodiment. In addition, another configuration may be added to, deleted from or replaced with part of one configuration of each embodiment.

In addition, each configuration, each function, each processing unit, each processing means and so forth which are aforementioned may be realized in hardware by designing some or all of them, for example, by an integrated circuit and doing something like that, and the processor interprets and executes the programs which realize the respective functions of them and thereby they may be realized in software.

Information on the program, the table, the file and so forth which realize respective functions can be stored into storage devices such as a memory, a hard disc, an SSD (Solid State Drive) and so forth or recording media such as an IC (Integrated Circuit) card, an SD card, a DVD (Digital Versatile Disc).

In addition, control lines and information lines which are thought to be necessary for description are shown and they do not necessarily show all the control lines and information lines which are necessary for implementation. In actuality, it may be thought that almost all the configurations are mutually connected.

## Claims

1. A data processing apparatus having:
a storage unit which stores an analysis target data group which has values of respective variables in a variable group and a value of an objective variable per analysis target and an element group that the variable group and each of one or more modulation method(s) for modulating the variable(s) are set as elements;
a modulation unit which when the element which is selected from the element group is acquired, plans a modulation function of modulating the value of the variable which is contained in the acquired element on the basis of an action history which is the history of the acquired element and modulates the value of the variable per the analysis target on the basis of the modulation function; and
a generation unit which generates image data which gives a point of coordinates which are values of the modulation result and the objective variable to a coordinate space which is defined by a first axis which corresponds to a result of modulation by the modulation unit and a second axis which corresponds to the objective variable per the analysis target.

2. The data processing apparatus according to claim 1 having:
a control unit which generates a control signal for selecting the element from the element group on the basis of the action history and controls the modulation unit on the basis of the control signal.

3. The data processing apparatus according to claim 2, wherein
when the image data is generated by the generation unit, the control unit selects the element from the element group and newly generates the control signal.

4. The data processing apparatus according to claim 3, wherein
the control unit defines the image data as a first state and defines the element group as a first action, inputs the image data into a first action value function which outputs a value of the first action in the first state on the basis of a first learning parameter, selects a specific element which corresponds to a specific value in the element-based values which are output from the first action value function from the element group, newly generates the control signal which contains the selected specific element and thereby controls the modulation unit.

5. The data processing apparatus according to claim 4, wherein
the specific value is a value which indicates a maximum value in the element-based values.

6. The data processing apparatus according to claim 4, having:
an accumulation unit which accumulates the image data by the generation unit; and
an evaluation unit which evaluates the modulation function on the basis of the modulation result and the value of the objective variable, wherein
the control unit selects a combination of time-series first image data and second image data which are accumulated from the accumulation unit, defines the image data as a second state and defines the element group as a second action, inputs the second image data into a second action value function for outputting a value of the second action in the second state on the basis of a second learning parameter, adds a result of evaluation of the modulation function by the evaluation unit to a second output result from the second action value function as a reward and thereby calculates a value of the first action as a teacher signal, updates the second learning parameter on the basis of the teacher signal and a first output result which is output in a case where the first image data is input into the second action value function and updates the first learning parameter by the updated second learning parameter.

7. The data processing apparatus according to claim 1, having:
an evaluation unit which evaluates the modulation function on the basis of the values of the modulation result and the objective variable; and
an output unit which outputs the image data to be displayable in a case where a result of evaluation of the modulation function by the evaluation unit is more than a target value.

8. The data processing apparatus according to claim 6, wherein
the objective variable is a quantitative variable, and
the evaluation unit generates a regression model for regressing the first-axis coordinate value which corresponds to the modulation result with the objective variable and outputs the accuracy of the regression model as a result of evaluation of the modulation function.

9. The data processing apparatus according to claim 6, wherein
the objective variable is information for classification of the analysis target data group, and
the evaluation unit generates an identification model which identifies the first-axis coordinate value which corresponds to the modulation result with the objective variable and outputs the accuracy of the identification model as a result of evaluation of the modulation function.

10. A data processing method that a data processing apparatus which has a processor which executes a program and a storage device which stores the program executes, wherein
the data processing apparatus is accessible to an analysis target data group which has values of respective variables in a variable group and a value of an objective variable per analysis target and to an element group which defines the variable group and each of one or more modulation method(s) for modulating the variable(s) as elements,
the processor executes
a modulation process of, when the element which is selected from the element group is acquired, planning a modulation function for modulating a value of the variable which is contained in the acquired element on the basis of an action history which is the history of the acquired element and modulating the value of the variable per the analysis target on the basis of the modulation function, and
a generation process of generating image data which gives a point of coordinates which are values of the modulation result and the objective variable to a coordinate space which is defined by a first axis which corresponds to a result of modulation by the modulation process and a second axis which corresponds to the objective variable, per the analysis target.

11. A data processing program for making a processor which is accessible to an analysis target data group which has values of respective variables in a variable group and a value of an objective variable per analysis target and to an element group which defines the variable group and each of one or more modulation method(s) of modulating the variable(s) as elements
execute
a modulation process of, when the element which is selected from the element group is acquired, planning a modulation function for modulating a value of the variable which is contained in the acquired element on the basis of an action history which is the history of the acquired element and modulating the value of the variable per the analysis target on the basis of the modulation function, and
a generation process of generating image data which gives a point of coordinates which are values of the modulation result and the objective variable to a coordinate space which is defined by a first axis which corresponds to a result of modulation by the modulation process and a second axis which corresponds to the objective variable, per the analysis target.
